# EUROPEAN PATENT APPLICATION

(11) **EP 4 062 878 A1**
(43) Date of publication of application: **28.09.2022**
(21) Application number: 22425013.4
(22) Date of filing: 18.03.2022
(51) Int. Cl.: A61F 2/54, A61F 2/58

(54) **MULTIFUNCTIONAL UPPER LIMB PROSTHESIS**

(30) Priority: 25.03.2021 IT 202100007337
(71) Applicant: Biodismed s.r.l, 00071 Pomezia (RM) (IT)
(72) Inventor: MAGGIORI, Roberto, 00071 Pomezia (RM) (IT)

(57) **Abstract**

The present invention relates to an upper limb prosthesis (1), comprising a body (2), to replace at least an amputated portion of a forearm of a patient; motor means (7) arranged inside said body (2) and can be operated from the outside; means of power supply (5), to power electrically said motor means (7); and means of coupling (9; 16), to couple in a removable said motor means (7) with at least one power tool (100; 200). The present invention also concerns a power tool (100; 200) which can be coupled to this prosthesis (1).

## Description

The present invention concerns a prosthesis of a multifunctional upper limb.

More precisely, the present invention relates to an active trans-radial prosthesis, capable of being coupled with power tools. Specifically, the present invention relates to a trans-radial prosthesis for power tools interchangeable with magnetic coupling.

The field of application of the present invention is therefore the medical field of devices aid for the disabled, and more precisely in the field of upper limb prostheses intended for the patient amputee.

In the state of the art, different types are known of active type upper-limb prosthesis, that is prostheses capable of being operated by the patient to perform at least one mechanical movement, such for example the taking of an object and/or the flexion/extension of the forearm on the arm.

Among the prostheses of the active type are therefore including both the prostheses that can be operated by pushing up special buttons with the contralateral limb that prostheses controlled by the electromyographic signals of the patient. In particular, such signals electromyography can be detected using the use of external electrodes placed on the skin patient or with implanted internal electrodes inside the patient. There are also active upper limb prostheses of the hybrid type on the market, which have both types of control, with external buttons and signal detection electromyography.

Indeed, in recent years the research has focused on human-machine interface methodology for controlling the movements of one hand bionics through myoelectric impulses of the patient.

In particular, bionic hands were studied that they could reproduce as faithfully as possible the movements of the joints of the phalanges and more in general about the grasping ability of a real hand.

However, currently, the amputee patient is not able to safely use tools for working in the hobby and/or professional field, and in particular electric tools, such as the example drills for drilling, orbital sanders for sanding, angle grinders to grind, the disc miter saws for cutting, screwdrivers electric to tighten bolts and/or threaded screws, etc..

This lack of research and development for this use, today appears completely unjustified, given that the amputee patient, like the subject able-bodied, has the same needs as use many tools of daily use. Often, the amputee patient is forced to use standard power tools with the use of the contralateral limb only, thus failing operational safety, as well as the handling normally resulting from the possibility to use power tools with both upper limbs.

It is therefore the specific purpose of this invention to overcome the disadvantages of a known technique.

In particular, the object of the present invention is to allow the amputee patient to use power tools in safety.

Another object of the present invention is to allow the amputee patient to return completely autonomous in the hobby and/or professional works.

In particular, the object of the present invention is to provide an upper limb prosthesis that allows amputee patient to use power tools safely, leaving free to cooperate with the contralateral limb.

Finally, the object of the present invention is to provide an upper-limb prosthesis, particularly a trans-radial prosthesis, which is safe and reliable.

These and other purposes have been achieved with the upper limb prosthesis according to the present invention, in which the amputated limb becomes one with the electric tool, leaving free to cooperate with the patient's contralateral upper limb.

Therefore, the object of the present invention is one upper-limb prosthesis, comprising
a body, to replace at least a portion amputation of a patient's forearm;
motor means arranged inside said body e operated from the outside;
electrical power supply means, for powering electrically said motor means; and
the coupling means, for coupling in a manner removable, said motor means with at least one power tool.

In particular, according to the invention, the prosthesis can comprise an electric tool coupled to the said body through said coupling means.

Furthermore, said coupling means can comprise a first connector disposed on one end of said body, said first connector defining a first housing, preferably of a substantially truncated cone shape.

Said coupling means can comprise also a second connector arranged on one end of the said electric tool and configured to fit into interlocking in the said first housing.

Specifically, according to the invention, inside said first housing can be arranged a first joining element, connected to said motor means, for transmitting, in use, a motion rotating from said motor means to said power tool.

Furthermore, according to the invention, said second connector may comprise a second element of the junction, complementary to said first element of the junction.

According to the invention, inside the said a first housing can be arranged electromagnet connected to said power supply means, and said second connector may include at least a portion made of ferromagnetic material, in such that, in use, said the first connector of the said body can be coupled stably with the said second connector of the said electric tool through the generation of a magnetic field.

In particular, according to the invention, said prosthesis may include a three-position switch connected to said motor means, to said means of power supply and to the said electromagnet, in wherein said switch can be configured in such a way such that two positions of said three positions can activate or switch off said motor means and a third one position can allow the coupling and the detachment of said second connector of said electric tool to/from said the first connector of said body, through the activation-deactivation of the said electromagnet.

Furthermore, according to the invention, said means of coupling may comprise at least one device safety connected to said motor means and to said means of power supply and configured for stop, in use, said motor means in the case of twisting of the said electric tool on the said body by disconnecting said motor means from said means of power supply.

In particular, according to the first invention connector can comprise said device of safety, and said the second connector can comprise a second housing to the house at least one portion of said safety device.

Furthermore, according to the invention, said device security can include
a cylindrical casing comprising a base front and a housing, accessible from the said base front, in which said housing ends in correspondence of an internal wall, substantially parallel to said front base;
a ring in abutment with said front base, using an abutment organ, and in turn housed in the said cylindrical casing;
a first electrode arranged at the said inner wall;
a second electrode disposed on the said ring;
a mobile contact, arranged inside said housing and having a rest configuration, in which it is in contact with the said second electrode and an operating configuration in which it is spaced from said second electrode;
a conducting element, arranged inside said housing, to electrically connect said contact movable with said first electrode;
a spring arranged in the said housing, to put a contrasting said movable contact with said wall internal; and
a sphere housed in said first connector in to protrude at least in part from it, being coupled to said movable and combinable contact, in use, with said second housing, in such a way that following a twisting of the said electric tool on said body, said sphere is compressed on said second housing resulting in compression of said spring and displacement of said movable contact in said operating configuration, to interrupt the electrical connection between said moving contact e said second electrode, and, consequently, between said one's motor means and said electrical power supply means.

It is a further object of the present invention a power tool in accordance with prosthesis object of the invention.

The invention will now be described for illustrative purpose but not limiting, with particular reference to the drawings of the attached figures, in which:
figure 1 shows an isometric view of a prosthesis according to the present invention comprising a body coupled to a first electric tool of the type screwdriver/drill through coupling means comprising a female connector of the body of the prosthesis and a male connector of the power tool;
figure 2 shows an exploded isometric view of the prosthesis of figure 1;
figure 3 shows an isometric view of the prosthesis of figure 1 in which the body is coupled to a second power tool of the grinder type angular;
figure 4 shows an isometric view of the body of the prosthesis of figure 1;
figure 5 represents a detail of figure 4, where the female connector of the means is shown coupling;
figure 6 shows an exploded isometric view of the female connector of the coupling means of the prosthesis of figure 1, in which the female connector includes a safety device;
figure 7 shows an exploded isometric view of the safety device of the female connector of figure 6;
figure 8 shows a front view of the safety device of figure 7, in which it is defined as an AA cutting plan;
figure 9 shows a sectional side view along the cutting plane AA of the device safety of figure 8;
figure 10 shows a sectional view of the coupling means of the prosthesis of figure 1, comprising the female connector of the body of the prosthesis and the male connector of the power tool;
figure 11 shows an isometric view of the angle grinder type power tool shown in figure 4;
figure 12 shows a side view of the power tool in figure 11; and
figure 13 is a detail of figure 11, in which the male connector of the means is shown coupling for power tools of the power tool in figure 11.

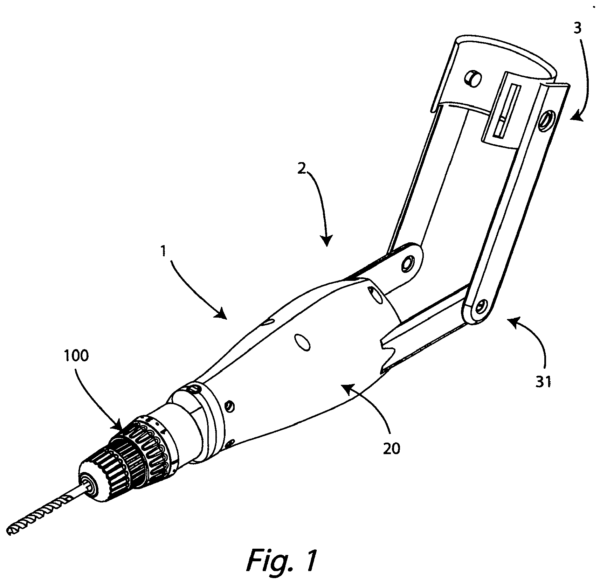

With particular reference to figures 1 - 3, is described an upper limb prosthesis 1 of the active type according to the present invention, comprising a body 2 removable coupled to an electric tool 100; 200 through coupling 9; 16. In particular, prosthesis 1 of the figure is a prosthesis for a patient to whom it has been amputated at least a portion of the forearm (trans-radial amputation) and body 2 replaces this amputated portion of the patient's forearm.

Body 2 can also be coupled to a hand prosthesis (not shown) through the same coupling means 9, 16. In particular, the hand prosthesis can be both aesthetic and one bionic hand.

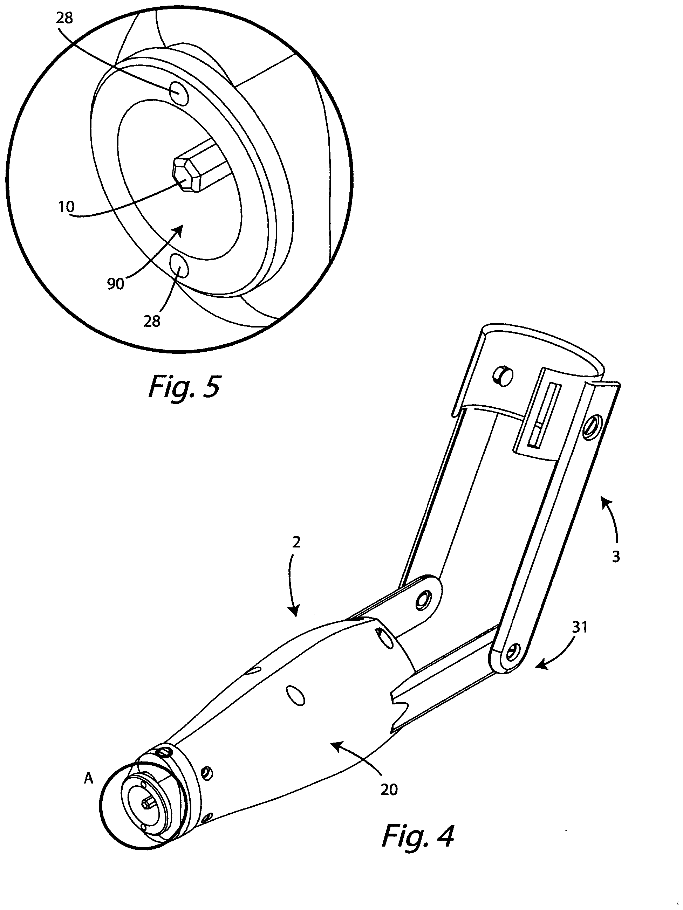

With particular reference to figures 1-4, the body the prosthesis 1 comprises
a support 3 for the patient's stump, and
a casing 20, coupled to support 3 through a joint 31.

Holder 3 is configured to pair with the patient's arm above the elbow.

In particular, support 3 is a support comprising a vertical portion 30 and a containment part 4, to contain the stump. The containment part 4 has a half-ring shape.

In alternative embodiments (not shown), prosthesis 1 can also be a prosthesis transhumeral, to replace the forearm and elbow of a patient who also had one amputated portion of the humerus. In this case, the coupling between support 3 and casing 20 can be of the type mechatronic, to allow the flexion/extension of the forearm concerning the stump. Such a mechatronic link in replacement of the elbow joint can be for example based on the electromyographic signals of the stump or it can be operated by acting on a special one switch.

As mentioned, casing 20 is coupled to support 3 through joint 31 to have freedom of rotation between casing 20 and support 3, at least to allow the movement of flexion/extension of casing 20 concerning the stump of the patient by moving the patient's elbow. As for the movement of pronation/supination, in a form of realization, this can be compensated for, at least partly by the possibility of the hand and/or of the power tool 100, 200 to assume positions different than the casing 20.

The casing 20 has a shape and size similar to the portion of the forearm removed.

Inside the casing 20 are housed motor means 7, in particular, a gearmotor electric 7, for the operation of power tools 100, 200; and
a battery 5, in particular a battery 5 of rechargeable type, for powering motor means 7.

Electrical connection means 6, in particular, an electronic control and power circuit 6, electrically connect the battery 5 with the means motors 7.

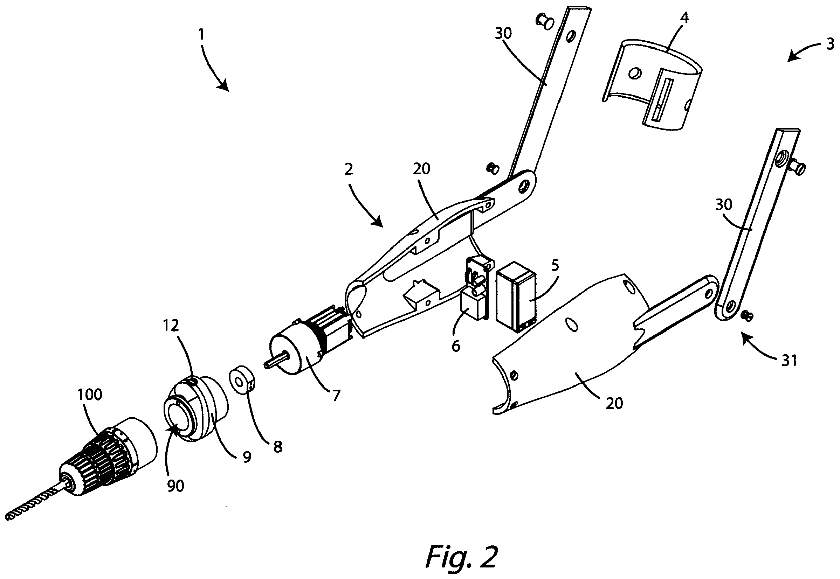

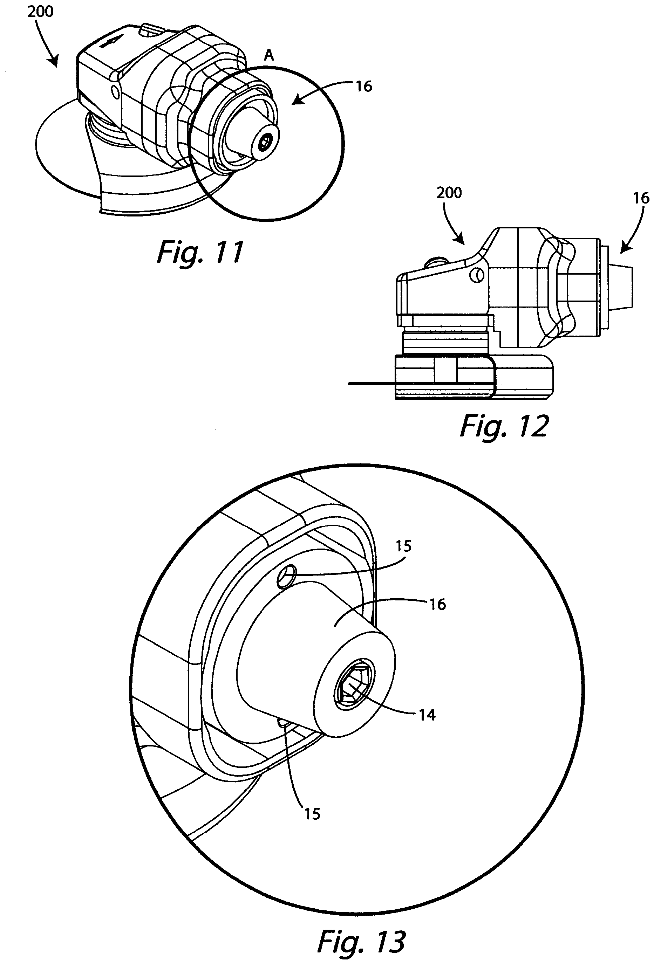

With particular reference to figures 2, 5, 6, and 10-13, the coupling means 9, 16 are arranged in correspondence to an anterior end of the prosthesis 1, being placed at the height of the missing wrist of the patient. Therefore, preferably, the forms, dimensions, and proportions of such coupling mean 9, 16 are similar to those of the patient's wrist.

In particular, the coupling means 9, 16 shown in the figures comprise a first connector 9 on one end of the body 2 and a second connector 16 on one end of the power tool 100; 200.

The second connector 16 can be coupled to the first connector 9 through interlocking coupling. The interlocking coupling shown is, in fact, a conical coupling, in particular truncated cone, wherein the first connector 9 is a connector female and the second connector 16 is a connector male.

This technical solution allows to obtain adequate friction between the coupling means 9, 16, necessary to guarantee the operational safety of the power tools inserted into the prosthesis. Also, like better illustrated below, this coupling is configured in such a way that upon passing a determined torque threshold is determined the immediate intervention of special devices safety 13, which blocks the motor electric 7.

This twisting moment can, for example, develop during the use of certain power tools that generate opposing torsional forces, with consequent slippage which involves a rotation between connectors 9, 16.

In particular, the first connector 9 defines a first housing 90 for receiving the second connector 16 and inside which are arranged:
a first joining element 10, in particular, a male joint 10 of substantially shape hexagonal, connected to the motor means 7, for transmitting the rotary motion to the power tool 100, 200; and
an electromagnet 8, placed inside the first connector 9 to allow coupling electromagnetic between body 2 of prosthesis 1 and the power tool 100, 200, especially between the first connector 9 of body 2 and the second connector 16 of the power tool 100, 200, and to guarantee the operation.

The first connector 9 further comprises an electric switch 12, connected to the means of power supply 5 and/or to the electrical connection means 6, and to the motor means 7 and to the electromagnet 8, to activate/deactivate the power supply of the motor means 7 and to allow the attachment/detachment of power tools 100, 200 through activating/deactivating of the electromagnet 8.

The electrical switch 12 is accessible from the outside and is preferably a three-position switch.

In fact, thanks to the three-position switch are possible:
- allow the coupling/uncoupling of the first connector 9 of the prosthesis 1 with the second connector 16 of the power tool 100, 200, through the activation/deactivation of the electromagnet 8 which generates a magnetic field to hold at least one metal portion (or other ferromagnetic material) of the power tool; and
- supply voltage to the motor means 7 for operating the power tool 100, 200 connected to the prosthesis.

In particular, the second connector 16 comprises at least a portion made of ferromagnetic material, in particular metal, to ensure coupling between the two connectors 9, 16 through activating the electromagnet 8. In the embodiment described, this portion in a ferromagnetic material is the flat surface of the truncated-cone shape which constitutes the second connector 16 of the male type.

Thanks to the particular conformation of the means of coupling 9, 16, the power tool 100, 200 become integral with the body 2 of the prosthesis 1, by the effect of the magnetic field generated by the electromagnet 8, in so that this configuration allows transmission of the rotary motion from the 7 motor means placed inside of the prosthesis 1, to the power tool 100, 200.

The first connector 9 also comprises two 13 safety devices to allow the safe coupling between prosthesis 1 and the power tool 100, 200.

In particular, these safety devices 13 are two electrical safety contacts 13, arranged on two opposite sides of the first connector 9, to ensure the safety of the coupling between the body 2 of prosthesis 1 with the second connector 16 of the power tool 100, 200.

In fact, safety devices 13 are normally closed electrical switches, configured to deactivate motor 7 when this occurs even a partial rotation of the power tool 100, 200 on the conical seat 90 of the connector 9, as best illustrated below.

Mirror, the second connector 16 on the power tool 100; 200 includes a second junction element 14, complementary to the first junction element 10.

In particular, the second connector 16 comprises a female joint 14 of substantially hexagonal-shaped, to transmit drive from the first element of junction 10 to electric tools 100; 200.

The second connector 16 further comprises a second housing 15 for each device of safety 13.

The hexagonal shape of the joints 14, 10 was specially designed to facilitate the coupling between electric tools 100, 200, and prosthesis 1.

Furthermore, the safety device 13 is configured to allow the immediate shutdown of the motor means 7, in case of a partial rotation of the power tool 100, 200 around the tapered seat of the first connector 9.

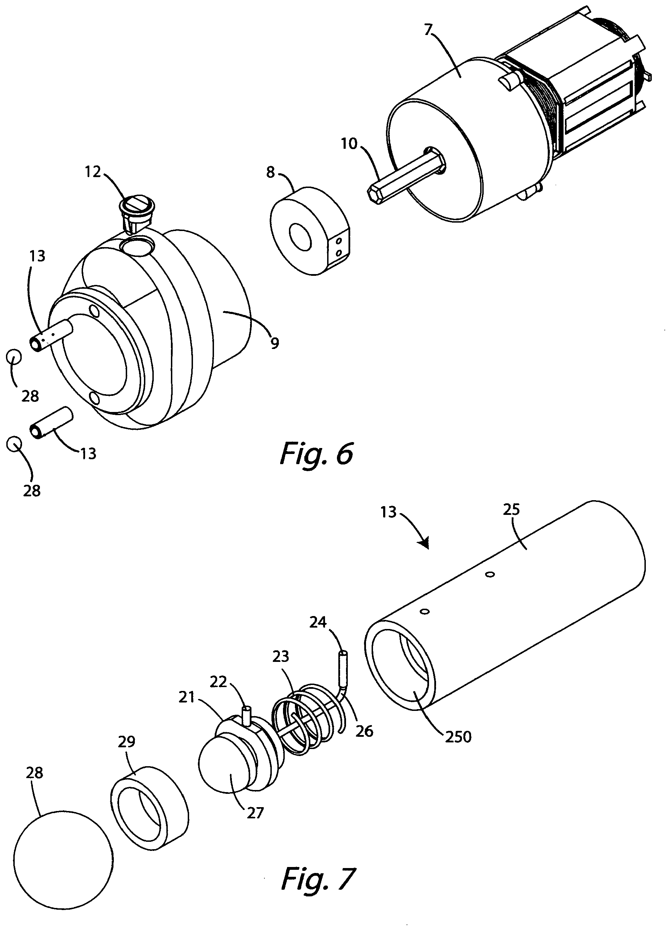

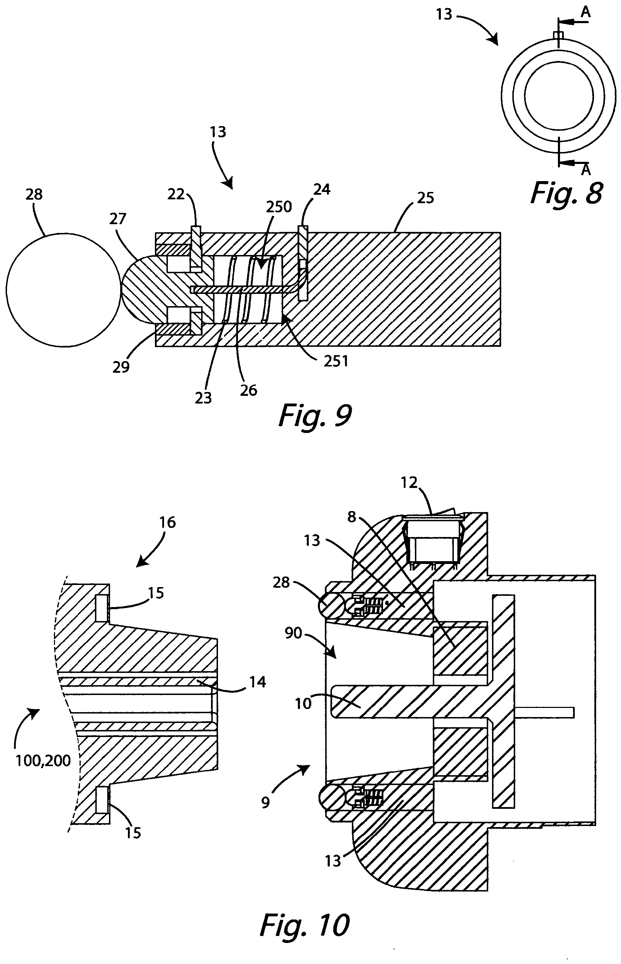

With particular reference to figures 7 - 9, each safety device 13 on the first connector 9 includes, in fact,
a cylindrical casing 25, comprising a base front and a seat 250, accessible from the anterior base and ending at a internal face 251 substantially parallel to the base front;
a ring 21, in particular a peripheral ring 21, abutting with an abutment element 29, housed in the cylindrical casing 25, which is better illustrated subsequently;
a first electrode 24, or electrical terminal rear 24, arranged inside the casing cylindrical 25, in correspondence with the internal face 251;
a second electrode 22, or electrical terminal front 22, arranged on the ring 21;
a mobile contact 27, arranged inside the seat 250, and configured to assume a configuration of rest in which it is in contact with the second electrode 22 and spaced from the first electrode 24;
said abutment element 29, in particular one front bushing 29, to keep in position the moving contact 27;
a conductive element 26, in particular a wire conductor 26, arranged inside the seat 250, to electrically connect the moving contact 27 with the first electrode 24;
a spring 23 placed in seat 250, in contrast between the internal face 251 of the cylindrical casing 25 and the moving contact 27; and
a sphere 28, housed in the connector 9 (such as shown in fig. 10) so as to protrude at least partially from it, being coupled to the movable contact 27 and inserted, in use, in the second housing 15 of the second connector 16.

As said, the safety device 13 serves a keep the coupling safe of the power tool 100, 200 to the body 2 of the prosthesis 1.

In fact, it allows protection that acts to the electrical stage, cutting off the voltage to the motor if a phenomenon of seizure, such as can occur when the drill bit 100 hooks to the burr that you shape at the exit of a hole. If such safety device 13 is not present, engine 7 would continue to provide rotary motion to the drill bit 100, creating a dangerous force of torsion that would first be transmitted to the prosthesis 1, until it affects the stump of the patient who performs the function of supporting the same prosthesis 1, with the consequent risk of serious injuries.

Therefore, if due to exceeding a certain threshold of torsion force occurs a rotation of the power tool 100, 200 concerning first connector 9, due to the interference mechanics that would be created between housing 15 and sphere 28 placed in front of the safety 13, the compression of the sphere 28, which, as mentioned, is freely housed in the second housing 15 of the second connector 16.

The sphere 28 would undergo a consequent advance axial, which acts directly on the moving contact 27, cutting off the electric current between the two electrodes 22, 24.

In particular, the power off occurs through the opening of the movable contact 27, which passes from a rest state in which it connects the first one electrode 24 with the second electrode 22, at one operative state in which such connection electrical is interrupted.

In fact, the movable contact 27 is normally placed in contact with the peripheral ring 21 equipped with electrode 22 (as visible in fig. 9) and is connected through a wire conductor 26 to electrode 24.

Therefore, in the rest state, namely in normally closed contact, the ring peripheral 21 equipped with electrode 22 is placed in contact with the moving contact 27, which conducts current to electrode 22, allowing the operation of the electric circuit that powers the electric motor 7.

Contrary, in case of intervention of the safety device 13 following pressure exerted by the advancement of the sphere 28 on the movable contact 27, said movable contact 27 retracts determining the opening of the electrical circuit, resulting in interruption of the electric current that powers the electric motor 7.

In other words, the safety devices 13 are normally closed electrical switches, configured to interrupt electric current to the electric motor 7 when there is a rotation, even partial, of the power tool 100, 200 on the conical seat 90 of connector 9, keeping in mind that by the effect of the interference that is created between the housings 15 created into connector 16 and the sphere 28 placed in front of the safety devices 13 an in turn housed in connector 9, the spheres 28 are pushed inwards by activating the electric switch that interrupts electric current that powers the electric motor 7, stopping it.

As for the power tools 100, 200, these can be any power tool suitable for hobby and/or professional activities, such for example drills for drilling, sanders orbitals for sanding, angle grinders to grind, the disc miter saws to cut, the electric screwdrivers to tighten bolts and/or screws threaded, etc..

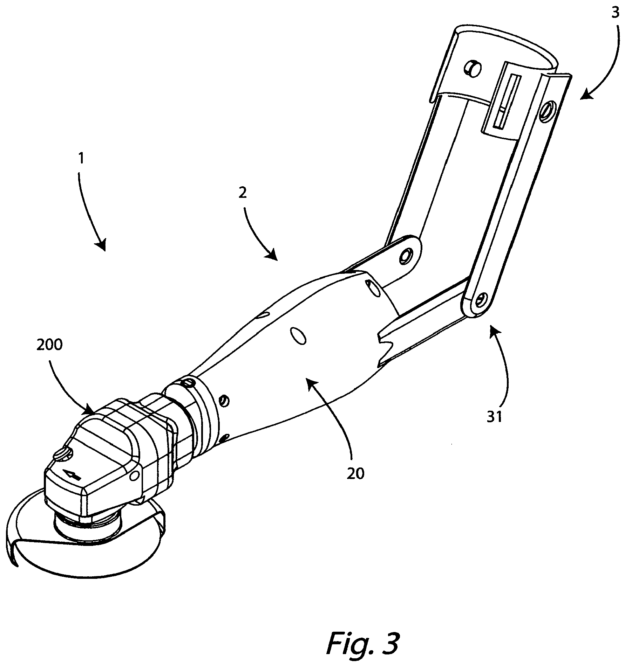

In the particular embodiments shown, body 2 of the prosthesis 1 is coupled to a power tool type screwdriver/drill 100, suitable for drilling various materials and the tightening screws and bolts (as shown in figures 1 and 2) or of the angle grinder type 200/200 miter saw, suitable for grinding and cutting metals (as shown in Figures 3 and 11-13).

In the case of the power tool of the type screwdriver/drill 100 the possible rotation of the power tool 100 on the body 2 of the prosthesis 1 and consequent activation of the safety device 13 for example, it can be caused by the seizure of the drill bit in the material being processed.

Thanks to the prosthesis 1 just described is, therefore, possible to guarantee to the amputee patient, optimal handling of power tools as well high operational safety.

Furthermore, the prosthesis 1 object of the present invention is simple to manufacture, as the means motors 7 and battery 5 can be motors electric and rechargeable batteries common to many power tools.

This technology, therefore, offers the advantage of using a universal prosthesis, which through an electromagnetic quick coupling that implements a safety device against injuries, allows the amputee patient to perform the work, becoming completely autonomous.

In what precedes the preferred embodiments and have been suggested of the variants of the present invention, but it is from it is understood that those skilled in the art will be able to contribute modifications and changes without thereby leaving the related scope of protection, as defined by claims attached.

## Claims

1. Upper limb prosthesis (1), comprising
a body (2), for replacing at least one amputated portion of a patient forearm;
motor means (7) arranged inside said body (2) and operable from the outside;
power supply means (5), for the power supply of said motor means (7); and
coupling means (9; 16), for coupling in a removable manner said motor means (7) with at least one power tool (100; 200).

2. Prosthesis (1) according to claim 1, **characterised in that** it comprises a power tool (100; 200) coupled to said body (2) by means of said coupling means (9; 16).

3. Prosthesis (1) according to claim 2, **characterised in that** said coupling means (9; 16) comprise a first connector (9) arranged on one end of said body (2), said first connector (9) defining a first housing (90), preferably with a substantially truncated-conic shape, and
**in that** said coupling means (9; 16) comprise a second connector (16) arranged on one end of said power tool (100; 200) and configured for being interlocked in said first housing (90).

4. Prosthesis (1) according to claim 3, **characterised in that** inside said first housing (90) it is arranged a first junction element (10), connected to said motor means (7), for transmitting, when in use, a rotational motion from said motor means to said power tool (100, 200); and
said second connector (16) comprises a second junction element (14), complementary to said first junction element (10).

5. Prosthesis (1) according to any one of claims 3 or 4, **characterised in that**
inside said first housing (90) it is arranged an electromagnet (8) connected to said power supply means (5), and **in that** said second connector (16) comprises at least one portion in ferromagnetic material, so that, when in use, said first connector (9) of said body (2) is steadily coupled with said second connector (16) of said power tool (100, 200) by generating a magnetic field.

6. Prosthesis (1) according to claim 5, **characterised in that** it comprises a three-position switch (12) connected to said motor means (7), to said power supply means (5) and to said electromagnet (8), wherein said switch (12) is configured so that two positions of said three positions turn on or turn off said motor means (7) and a third position allow the coupling and de-coupling of said second connector (16) of said power tool (100, 200) to/from said first connector (9) of said body (2), by activating/deactivating said electromagnet (8).

7. Prosthesis (1) according to any one of the preceding claims, **characterised in that** said coupling means (9; 16) comprise at least one safety device (13) connected to said motor means (7) and to said power supply means (5) and configured for halting, when in use, said motor means (7) in case of torsion of said power tool (100, 200) on said body (2) by disconnecting said motor means (7) from said power supply means (5).

8. Prosthesis (1) according to claim 7 and any one of claims 2 - 6, **characterised in that** said first connector (9) comprises said safety device (13) and said second connector (16) comprises a second housing (15) for housing at least one portion of said safety device (13).

9. Prosthesis (1) according to claim 8, **characterised in that** said safety device (13) comprises
a cylindrical casing (25) comprising a front base and a slot (250), accessible from said front base, wherein said slot (250) ends at an inner wall (251), substantially parallel to said front base;
a ring (21) abutting said front base, by means of an abutting unit (29), and which in turn is arranged inside said cylindrical casing (25);
a first electrode (24), arranged at said inner wall (251);
a second electrode (22), arranged on said ring (21) ;
a moving contact (27), arranged inside said slot (250) and having a resting configuration, in which it is in contact with said second electrode (22) and a working configuration in which it is apart from said second electrode (22);
a conductive element (26), arranged inside said slot (250), for the electrical connection of said moving contact (27) with said first electrode (24);
a spring (23) arranged inside said slot (250), for counteracting said moving contact (27) with said inner wall (251); and
a sphere (28) housed in said first connector (9) so as to protrude at least partially from it, being coupled with said moving contact (27) and coupleable, when in use, to said second housing (15), so that after a torsion of said power tool (100; 200) on said body (2), said sphere (28) is pressed on said second housing (15) with the consequent compression of said spring (23) and displacement of said moving contact (27) in said working configuration, so as to interrupt the electrical connection between said moving contact (27) and said second electrode (22), and, consequently, between said motor means (7) and said power supply means (5).

10. Power tool (100; 200) for a prosthesis according to any one of the preceding claims.
